# EUROPEAN PATENT APPLICATION

(11) **EP 4 650 356 A1**
(43) Date of publication of application: **19.11.2025**
(21) Application number: 25750267.4
(22) Date of filing: 28.02.2025
(51) Int. Cl.: C07D 491/052

(54) **PYRANO-PYRIDINE COMPOUND, PREPARATION METHOD THEREFOR, PHARMACEUTICAL COMPOSITION THEREOF AND USE THEREOF**

(30) Priority: 01.03.2024 CN 202410234745; 26.02.2025 CN 202510219066
(71) Applicant: Zhejiang Yangli Pharmaceutical Technology Co., Ltd., Hangzhou, Zhejiang 310015 (CN)
(72) Inventor: LIU, Yang, Shenzhen, Guangdong 518001 (CN); WU, Linlin, Shenzhen, Guangdong 518001 (CN); LI, Jianyong, Shenzhen, Guangdong 518001 (CN); ZHOU, Ji, Shenzhen, Guangdong 518001 (CN); ZHANG, Haolong, Shenzhen, Guangdong 518001 (CN); DING, Charles Z., Shenzhen, Guangdong 518001 (CN); FANG, Douglas, Shenzhen, Guangdong 518001 (CN); SHA, Wei, Shenzhen, Guangdong 518001 (CN)
(74) Representative: Müller-Boré & Partner Patentanwälte PartG mbB
(86) International application number: PCT/CN2025/079990
(87) International publication number: WO 2025/180518

(57) **Abstract**

The invention discloses a pyranopyridine compound, a preparation process, a pharmaceutical composition and use thereof. The present invention provides a compound of Formula (I), a pharmaceutically acceptable salt thereof, or a stereoisomer thereof, which has a strong inhibitory effect on aldosterone synthase, but has little effect on cortisol synthase, is highly selective and has a higher safety, and can be used for preventing and/or treating various diseases associated with aldosterone.

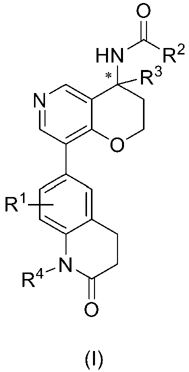

## Description

The present disclosure claims priorities of the Chinese patent application No. 2024102347458 filed on March 1, 2024, and the Chinese patent application No. 2025102190668 filed on February 26, 2025. The entireties of the above-mentioned Chinese patent applications are incorporated herein by reference.

### FIELD

The present disclosure belongs to the field of medicine, and particularly relates to a pyranopyridine compound, process for preparing the same, pharmaceutical composition and use thereof.

### BACKGROUD

Aldosterone is the renin-angiotensin-aldosterone system (RAAS), which is the core regulatory system of blood pressure and water-salt balance in the human body. Aldosterone is a key regulatory molecule downstream of the system. Aldosterone is a steroid hormone (mineralocorticoid family) that promotes renal re-absorption of water and sodium by binding and activating mineralocorticoid receptors (MR) in distal renal tubular and collecting tubular epithelial cells, while inducing excretion of potassium and hydrogen ions, maintaining balance between water and electrolytes, and participating in maintaining appropriate blood pressure, vascular tension and tissue perfusion. In addition, recent studies have shown that aldosterone can also upregulate ATIR expression on vascular smooth muscle cells, alter the tension of vascular smooth muscle, respond to contractile vascular signals and arterial wall structures, increase the vasopressor response of blood vessels to norepinephrine, and cause blood pressure elevation, vascular smooth muscle cell proliferation, vascular wall thickening and vitreous degeneration.

Under normal conditions, aldosterone concentration in plasma is regulated by relevant stimulus regulatory factors, such as RAAS, blood potassium concentration, and adrenocorticotropic hormone (ACTH). The elevated level of aldosterone can induce blood pressure disorders, lead to inflammation, vascular remodeling and tissue fibrosis associated with cardiac metabolic disorders, and ultimately lead to decreased organ function, cardiovascular complications, advanced kidney disease and increased risk of death. Therefore, combating the deleterious effects of excess aldosterone in patients has been a targeted clinical strategy for many years.

Blockade of the aldosterone effect is an effective treatment for cardiorenal diseases associated with aldosterone and its receptors. Mineralocorticoid receptor antagonists (MRA) and renin-angiotensin-aldosterone system antagonists (RAS inhibitors) are current clinical therapies for antagonizing aldosterone. MRA (e.g., spironolactone) inhibits aldosterone action by competitively binding to mineralocorticoid receptors, while RAS inhibitors (e.g., sartan) indirectly reduce aldosterone levels by blocking the upstream stimulation of angiotensin II. Clinically, MRA, on the one hand, excessively antagonizes receptor effects (aldosterone receptors may also be agonized by estrogen) with off-target side effects of androgen receptor antagonism, while on the other hand, RAS inhibitors do not completely inhibit excess aldosterone. There is a clinical resistance problem. Therefore, a specific inhibitor (ASI) that directly inhibits aldosterone synthase (AS) can completely reduce aldosterone production without additional effects and can serve as an optimal iterative product for MRA and RAS inhibitors.

Aldosterone synthetase (encoded by CYP11B2 gene) controls the synthesis of aldosterone and catalyzes the final step in the synthesis of aldosterone from cholesterol, which has been a pharmacological target for the treatment of hypertension for decades. Potassium ions, angiotensin II and leptin can activate the production of CYP11B2, thereby synthesizing aldosterone. Importantly, CYP11B2 is the only enzyme that catalyzes the final oxidation to aldosterone and is expressed primarily in the adrenal glomerular zone, which is not substantially produced elsewhere in the body and therefore is not expected to produce off-target effects.

Since 93% of aldosterone-producing enzymes and cortisol-producing enzymes are identical (CYP11B1, i.e. cortisol synthase, the final enzyme in the cortisol synthesis pathway), this high degree of similarity leads to cross-reaction and inhibition of cortisol synthesis by early aldosterone synthase inhibitors. Therefore, the development of a drug that inhibits aldosterone production without affecting cortisol is a current difficulty and pain point.

LCI699 is the first orally active aldosterone synthase inhibitor that entered into clinical trials for the treatment of primary aldosteronism. After oral administration of LCI699, a decrease in aldosterone level in plasma and a decrease in blood pressure were found. However, LCI699 is less selective for CYP11B2 and CYP11B1 and has a more inhibitory effect on cortisol synthetase, so it brings additional side effects and has to turn to the development of treatment for Cushing's disease. Since then, a new generation of highly selective ASI inhibitors is being developed and only a few products have entered the clinic up to now.

Lorundrostat (Mineralys) is a highly selective aldosterone synthase inhibitor that inhibits aldosterone synthase CYP11B2 and reduces aldosterone level *in vivo* without inhibiting CYP11B1. Another new drug is Baxdrostat (CinCor Pharma/AstraZeneca). The phase I clinical study on Baxdrostat has shown that Baxdrostat inhibits aldosterone synthase by 100 times as much as it inhibits cortisol synthesis and is a highly selective aldosterone synthesis inhibitor capable of dose-dependently reducing aldosterone level in plasma by more than 70%.

Although there are two clinical research products, it is unclear whether they ultimately prove safe and effective in large clinical Phase III. Therefore, highly selective aldosterone synthase inhibitors with good selectivity, higher safety and better efficacy are still desirable for patients.

### SUMMARY

The technical issue to be resolved by the present disclosure is to provide a novel aldosterone synthase inhibitor with high selectivity. The present disclosure aims to provide a pyranopyridine compound, process for preparing the same, pharmaceutical composition and use thereof. The compounds have strong inhibitory effects on aldosterone synthase, but have little effects on cortisol synthase, have high selectivity and high safety, and have good application prospects in preventing and/or treating various diseases related to aldosterone.

The present disclosure resolves the above technical issue by the following technical solutions.

The present disclosure provides a compound of formula (I), a pharmaceutically acceptable salt thereof, or a stereoisomer thereof, 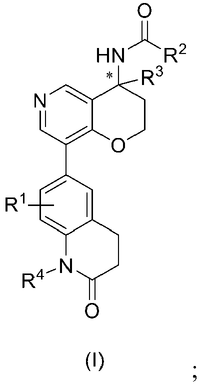 wherein carbon atom labeled by * represents S configuration, R configuration, or a mixture thereof;
R¹ is H, D, halogen, -CN, -NO₂, C₁-C₆ alkyl, C₁-C₆ haloalkyl, C₁-C₆ alkoxy or C₁-C₆ haloalkoxy;
R² is H, D, C₁-C₆ alkyl, C₁-C₆ alkyl substituted with 1, 2, or 3 R²⁻¹, C₁-C₆ alkoxy, C₁-C₆ haloalkoxy, 5- to 10-membered heteroaryl with 1, 2, or 3 heteroatoms selected from the group consisting of N, O, and S, 5- to 10-membered heteroaryl with 1, 2, or 3 heteroatoms selected from the group consisting of N, O, and S substituted with 1, 2, or 3 R²⁻¹, C₃-C₆ cycloalkyl or C₃-C₆ cycloalkyl substituted with 1, 2, or 3 R²⁻³ or NR²⁻⁴R²⁻⁵
R²⁻¹ is independently hydroxy or halogen;
R²⁻² and R²⁻³ are independently halogen;
R²⁻⁴ and R²⁻⁵ are independently H, C₁-C₆ alkyl or C₁-C₆ haloalkyl;
R³ is H, D, C₁-C₆ alkyl or C₁-C₆ haloalkyl;
R⁴ is C₁-C₆ alkyl.

In some preferred embodiments of the present disclosure, certain groups of the compound of Formula I, the pharmaceutically acceptable salt thereof, or the stereoisomer thereof are defined below, and unmentioned groups are as described in any one of the embodiments of the present disclosure ("in an embodiment of the present disclosure").

In an embodiment of the present disclosure, each C₁-C₆ alkyl and each C₁-C₆ alkyl in the substituted C₁-C₆ alkyl are independently methyl, ethyl, propyl, butyl or hexyl; preferably are methyl or ethyl.

In an embodiment of the present disclosure, each C₁-C₆ haloalkyl is independently halomethyl, haloethyl, halopropyl, halobutyl or halohexyl, the halo is fluoro, chloro, bromo or iodo.

In an embodiment of the present disclosure, each halogen is independently fluoro, chloro, bromo or iodo; preferably is fluorine.

In an embodiment of the present disclosure, each C₁-C₆ alkoxy is independently methoxy, ethoxy, n-propoxy, isopropoxy, n-butoxy, isobutoxy, sec-butoxy or tert-butoxy.

In an embodiment of the present disclosure, each C₁-C₆ haloalkoxy is independently halomethoxy, haloethoxy, halopropoxy, haloisopropoxy, halobutoxy, haloisobutoxy, halosec-butoxy or halotert-butoxy; the halo is fluoro, chloro, bromo or iodo.

In an embodiment of the present disclosure, each C₃-C₆ cycloalkyl and each C₃-C₆ cycloalkyl in the substituted C₃-C₆ cycloalkyl are independently cyclopropyl.

In an embodiment of the present diclosure, each 5- to 10-membered heteroaryl and each 5- to 10-membered heteroaryl in the substituted 5- to 10-membered heteroaryl are independently a 5- to 6-membered monocyclic heteroaryl.

In an embodiment of the present disclosure, each 5- to 10-membered heteroaryl and each 5- to 10-membered heteroaryl in the substituted 5- to 10-membered heteroaryl are independently selected from N, and the number of heteroatom is independently 1; for example

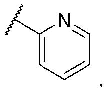

In an embodiment of the present disclosure, the carbon atom labeled by * represents R configuration.

In an embodiment of the present disclosure, R¹ is H or halogen.

In an embodiment of the present disclosure, R² is C₁-C₆ alkyl or NR²⁻⁴R²⁻⁵; preferably is C₁-C₆ alkyl.

In an embodiment of the present disclosure, R²⁻⁴ is H or C₁-C₆ alkyl; preferably is C₁-C₆ alkyl.

In an embodiment of the present disclosure, R²⁻⁵ is C₁-C₆ alkyl.

In an embodiment of the present disclosure, R¹ is H or fluoro.

In an embodiment of the present disclosure, R² is 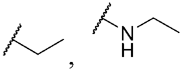 or 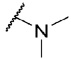 ; preferably is 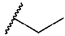 or 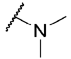 ; more preferably is

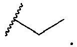

In an embodiment of the present disclosure, R³ is H or D; preferred is H.

In an embodiment of the present disclosure, said R⁴ is methyl.

In an embodiment of the present disclosure, the compound of formula (I) is a compound of formula (I-1) 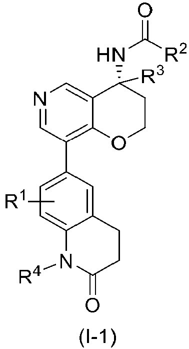 ; wherein R¹, R², R³ and R⁴ are as defined in any one embodiment of the present disclosure.

In an embodiment of the present disclosure, the compound of formula (I) is a compound of formula (I-2) 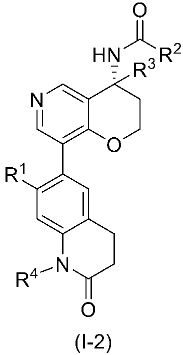 ; wherein R¹, R², R³ and R⁴ are as defined in any one embodiment of the present disclosure.

In some embodiments, the compound of formula (I) is any one of the following: 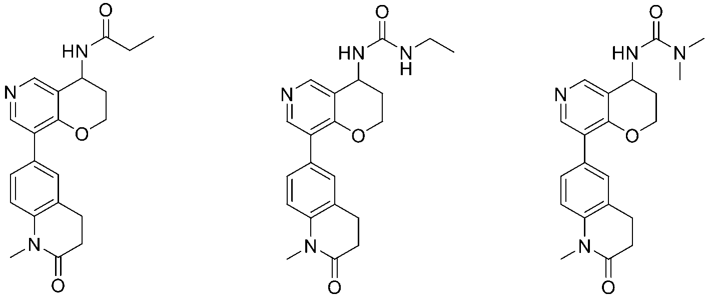 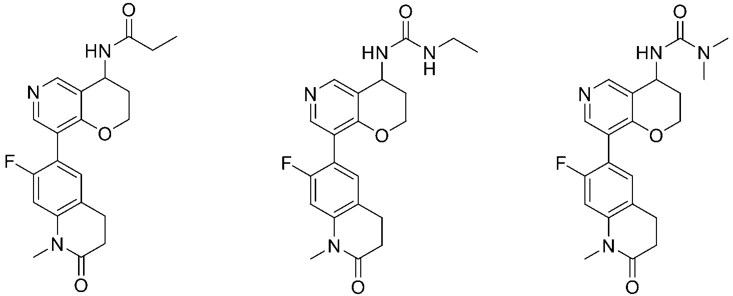 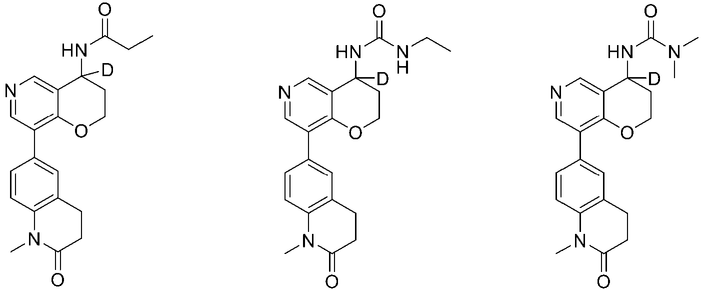 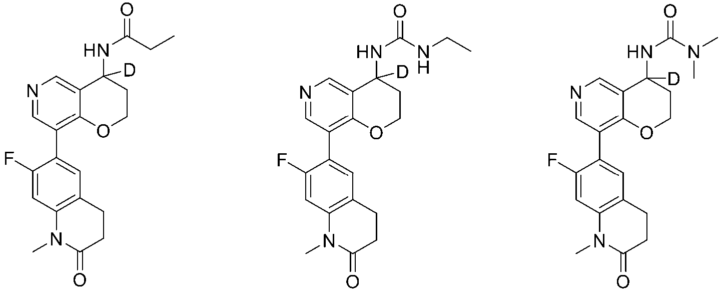 ; preferably is

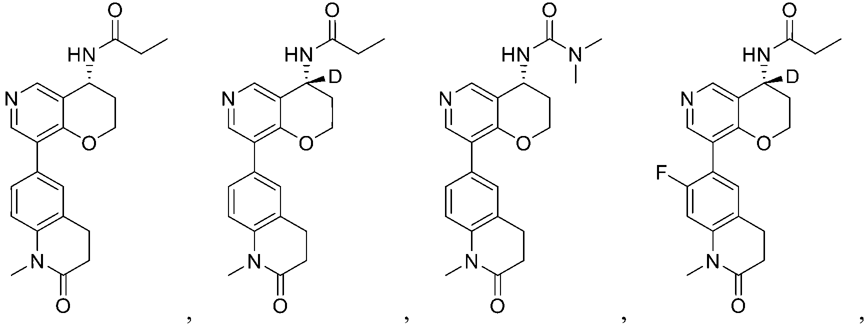

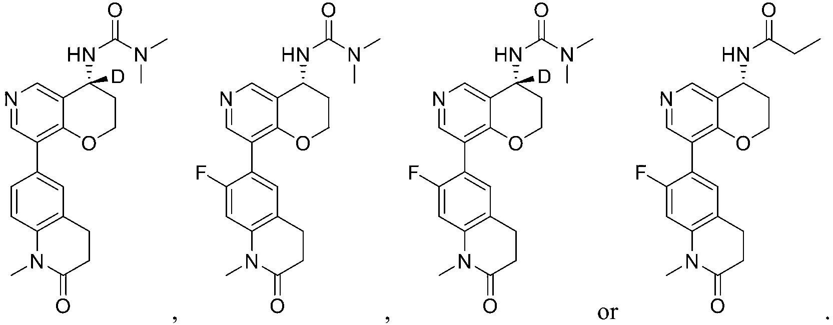

In some embodiments, the compound of formula (I) is not 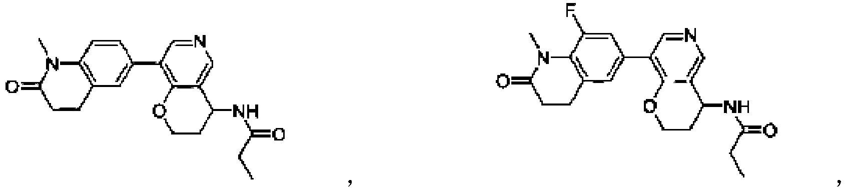 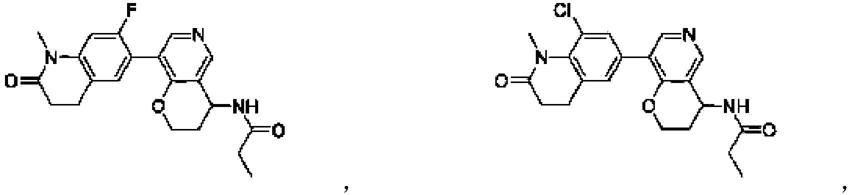 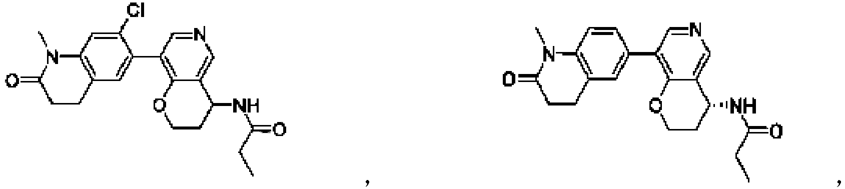 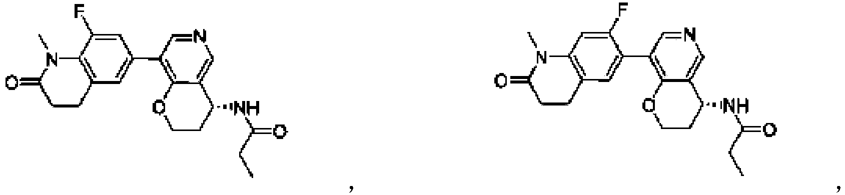 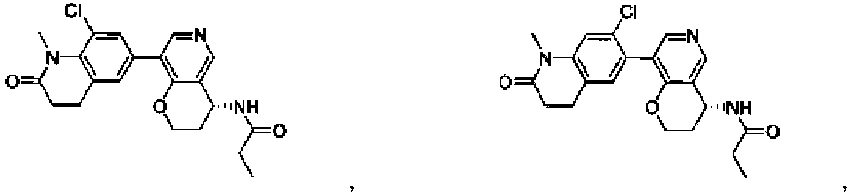 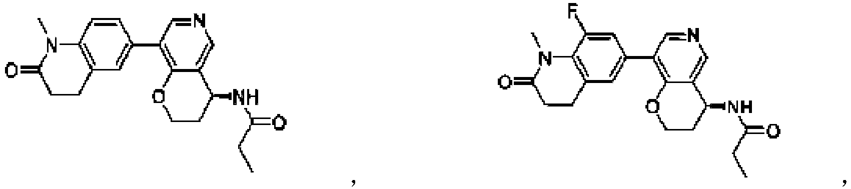 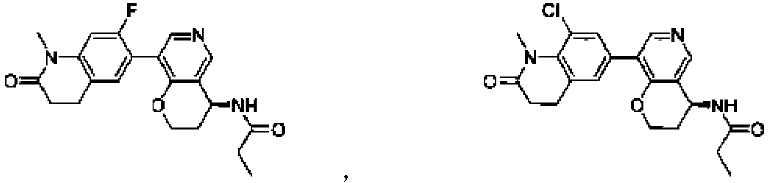 or

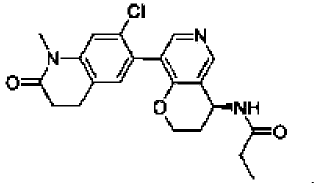

The present disclosure also provides a process for preparing the compound of formula (I), comprising preparing compound II and compound III in the presence of a base and a catalyst in a solvent to obtain the compound of formula (I); 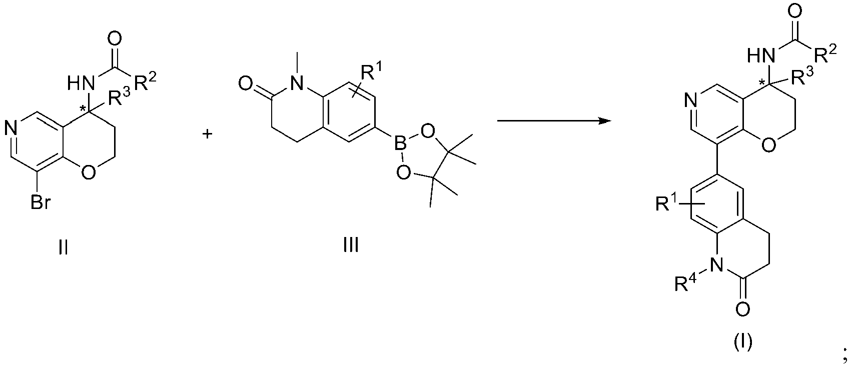 wherein *, R¹, R², R³ and R⁴ are as defined in any one embodiment of the present disclosure.

In some embodiments, the solvent is an organic solvent and/or water; preferably is an organic solvent and water; the organic solvent may be an alcohol solvent; for example, ethanol.

In some embodiments, the base is an inorganic base; preferably is potassium carbonate and/or sodium carbonate.

In some embodiments, the catalyst is a palladium catalyst; preferred is tetrakis(triphenylphosphine)palladium.

The present disclosure also provides a pharmaceutical composition comprising:
(1) The above compound of formula (I), the pharmaceutically acceptable salt thereof, or the stereoisomer thereof; and
(2) a pharmaceutically acceptable excipient.

The present disclosure also provides use of the compound of formula (I), the pharmaceutically acceptable salt thereof, or the stereoisomer thereof as described above, the pharmaceutical composition as described above in the medicament of an aldosterone synthetase inhibitor.

The present disclosure also provides use of the compound of formula (I), the pharmaceutically acceptable salt thereof, or the stereoisomer thereof as described above, the pharmaceutical composition as described above in the manufacture of a medicament for treatment and/or prevention of chronic kidney disease, congestive heart failure, hypertension or primary hyperaldosteronism; Preferably hypertension; preferably, the compound of formula (I) is not

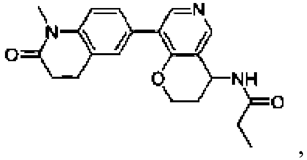

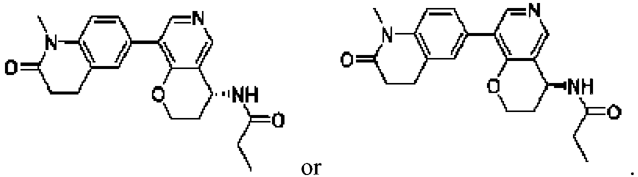

### Definition

Unless defined otherwise, all technical and scientific terms used herein have the same meaning as commonly understood by one skilled in the art to which the present disclosure pertains. In addition, the following definitions are set forth to illustrate and define the meaning and scope of the various terms used to describe the present disclosure.

The term "halogen" refers to fluorine, chlorine, bromine or iodine.

The term "alkyl" refers to a linear or branched alkyl group having the specified number of carbon atoms (e.g., C₁-C₆). Alkyl includes, but is not limited to, methyl, ethyl, n-propyl, isopropyl, n-butyl, tert-butyl, isobutyl, sec-butyl, n-pentyl, n-hexyl, and the like.

The term "haloalkyl" refers to a halogen-substituted alkyl, wherein halogen and alkyl are as defined above.

The term "alkoxy" refers to the group R^{Z}-O-, wherein R^{Z} is an alkyl group as defined above.

The term "haloalkoxy" refers to a halogen-substituted alkoxy group, wherein halogen and alkoxy are as defined above.

The term "heteroaryl" refers to a cyclic group having the specified number of ring atoms (e.g., 5- to 10-membered), the specified number of heteroatoms (e.g., 1, 2, or 3) and the specified heteroatom species (one, two, or three of N, O, and S), which is monocyclic or multicyclic, and each ring is aromatic (in accordance with the Huckel rule). Heteroaryl is attached to the rest of the molecule via a carbon atom or heteroatom. Heteroaryl is attached to the rest of the molecule via a ring with or without heteroatom. Heteroaryl includes, but is not limited to, furan rings, pyrrole rings, thiophene rings, pyrazole rings, imidazole rings, oxazole rings, thiazole rings, pyridine rings, pyrimidine rings, indole rings, benzopyrrole rings, and the like.

The term "cycloalkyl" refers to a saturated monocyclic cyclic group consisting solely of carbon atoms having the specified number of carbon atoms (e.g., C₃-C₆). Cycloalkyl includes, but is not limited to, cyclopropyl, cyclobutyl, cyclopentyl, cyclohexyl, and the like.

The term "pharmaceutically acceptable salt" includes "pharmaceutically acceptable salt formed with organic or inorganic acid" and "pharmaceutically acceptable salt formed with organic or inorganic base".

The term "stereoisomer" includes configurational isomer, wherein the configurational isomer primarily includes optical isomer, e.g., enantiomer, diastereomer, or a mixture thereof.

The term "pharmaceutically acceptable excipient" refers to any formulation or carrier medium capable of delivering an effective amount of an active substance of the present disclosure, which does not interfere with the biological activity of the active substance, and which has no toxic side effects on host or patient. Representative excipients include water, oil, vegetables and minerals, cream bases, lotion bases, ointment bases, and the like. These matrices include suspending agents, viscosity increasers, transdermal enhancers, and the like. Their formulations are well-known to one skilled in the cosmetic or topical pharmaceutical fields.

The term "pharmaceutical composition" refers to a mixture or solution comprising a therapeutically effective amount of an active pharmaceutical ingredient and a pharmaceutically acceptable excipient to be administered to a mammal, for example a human, in need thereof.

The term "treatment" refers to reversing, alleviating, inhibiting the progression or prevention of a disorder or condition to which the term applies, or one or more symptoms of such disorder or condition. As used herein, the term "treating" refers to the act of a treatment, which is as previously defined.

The preferred embodiments of the present disclosure can be obtained by any combination of the above-mentioned preferred conditions without departing from common knowledge in the art.

The reagents and raw materials used in the present disclosure are commercially available.

The positive technical effects of the present disclosure reside in that the present disclosure provides a pyranopyridine compound, process for preparing the same, pharmaceutical composition and use thereof. The present disclosure provides a class of selective aldosterone synthase inhibitors that have different structures from those of the reported or disclosed compounds. The compounds of the present disclosure have a selectivity factor for hCYP11B 1/2 of 50 to 220. The compounds of the present disclosure have excellent pharmacokinetic properties: aldosterone concentration in plasma at 12 hours after administration of the compound of the present disclosure is 85 to 220 pg/mL, corticosterone concentration in plasma at 12 hours after administration is 350 to 1000 pg/mL, maximum plasma concentration (Cₘₐₓ) is 8 to 25 ng/mL, and plasma exposure (AUC₀₋₂₄ₕ) is 150-2200 h·ng/mL. The *in vitro* inhibitory activity of the compounds of the present disclosure in connection with the common CYP450 enzyme is >50 µM and the compounds of the present disclosure have higher safety.

### BRIEF DESCRIPTION OF THE DRAWINGS

Fig. 1 shows the aldosterone concentrations in plasma at different time points after administration.
Fig. 2 shows the cortisol concentrations in plasma at different time points after administration.

### DETAILED DESCRIPTION

The present invention is further illustrated by way of example, but is not therefore limited to the scope of the described examples. Experimental methods not specified in the following examples are selected according to conventional methods and conditions, or according to the specification of the merchandise.

The starting materials or reagents used herein are commercially available or prepared by synthetic methods commonly known in the art.

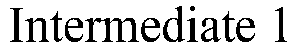

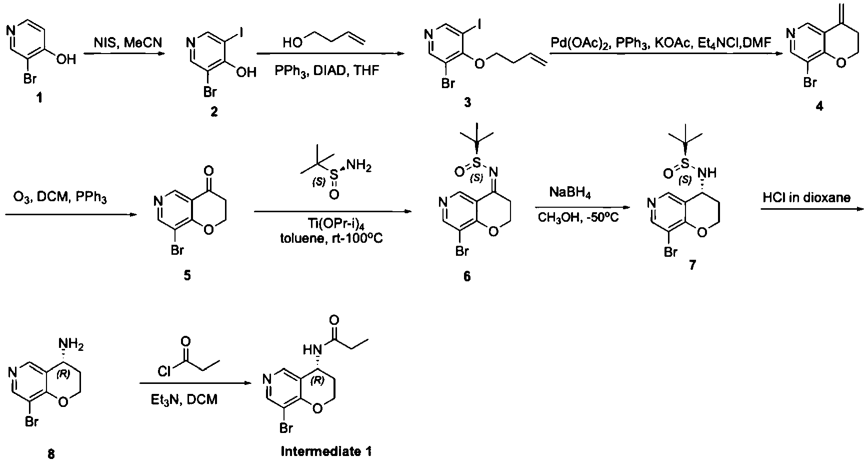

### Step 1

Compound 1 (30.0 g, 172.42 mmol) was dissolved in acetonitrile (300 mL). To the mixture was added N-iodosuccinimide (46.5 g, 206.90 mmol). After the addition, the reaction was stirred at 80°C for 2.5 hours. TLC (dichloromethane/methanol = 10/1, product: Rf = 0.1, starting materials: Rf = 0.2) showed complete consumption of the starting materials. The reaction solution was filtered while hot and washed with acetonitrile (200 mL). A solid was collected and dried to give compound 2 as a white solid (45.0 g, 87%).

LCMS (ESI) m/z: 299.8 [M+H]+.

### Step 2

Compound 2 (40.0 g, 133.38 mmol) in tetrahydrofuran (400 mL) were added 3-butene-1-ol (9.85 g, 136.60 mmol) and triphenylphosphine (42.0 g, 160.06 mmol). After the addition, the reaction solution was stirred at room temperature for 0.5 hour and cooled to 0°C. To the reaction sulution was slowly added diisopropyl azodicarboxylate (29.7 g, 146.72 mmol). After the addition, the reaction solution was stirred at 65°C for 12 hours under nitrogen atmosphere. TLC (petroleum ether/ethyl acetate = 10/1, product: Rf = 0.8, starting materials: Rf = 0.02) showed complete consumption of starting materials. The reaction solution was concentrated under reduced pressure and purified by column chromatography (petroleum ether/ethyl acetate = 20/1) to give compound 3 as a yellow oil (30.7 g, yield: 65%).

LCMS (ESI) m/z: 353.9 [M+H]+.

### Step 3

Compound 3 (30.7 g, 1.0 eq) was dissolved in N,N-dimethylformamide (300 mL). To the mixture were added triphenylphosphine (4.55 g, 17.35 mmol), tetraethylammonium chloride (14.4 g, 86.73 mmol), palladium acetate (1.95 g, 8.67 mmol) and potassium acetate (21.3 g, 216.82 mmol) successively. After the addition, the reaction solution was stirred under nitrogen atmosphere at 80°C for 1 hour. TLC (petroleum ether/ethyl acetate = 5/1, product: Rf = 0.4, starting materials: Rf = 0.78) showed complete consumption of starting materials. The reaction solution was cooled and then quenched with aqueous hydrochloric acid (300 mL, 4 M) to the reaction solution. The reaction solution was extracted with ethyl acetate (300 mL×2). The organic phase was washed with aqueous hydrochloric acid (200 mL, 4 M). The aqueous phase was collected. The pH of the aqueous phase was adjusted to more than 7 with 2 M aqueous sodium hydroxide. The aqueous phase was extracted with ethyl acetate (300 mL, 200 mL). The organic phase was washed with saturated sodium chloride solution (300 mL), dried over anhydrous sodium sulfate and filtered. The filtrate was concentrated under reduced pressure to obtain compound 4 as a yellow solid (20.0 g, yield: 76.5%).

LCMS (ESI) m/z: 226.0 [M+H]+.

### Step 4

Compound 4 (15.0 g, 1.0 eq) was dissolved in dichloromethane (600 mL). The mixture was cooled to -50°C under nitrogen atmosphere. To the mixture was pumped ozone. The temperature was kept. The mixture was stirred for 2 hours. TLC (petroleum ether/ethyl acetate = 4/1, product: Rf = 0.3, starting materials: Rf = 0.4) showed complete consumption of starting materials. The reaction solution was cooled to room temperature, quenched with saturated aqueous sodium sulfite solution (300 mL) and extracted by dichloromethane (200 mL×2). The organic phases were combined, washed with saturated aqueous sodium chloride solution (150 mL), dried over anhydrous sodium sulfate and filtered. The filtrate was concentrated under reduced pressure and purified by column chromatography (petroleum ether/ethyl acetate = 4/1) to give compound 5 as a yellow solid (13.0 g, yield 86%).

LCMS (ESI) m/z: 227.9 [M+H]+.

### Step 5

Compound 5 (3.0 g, 1.0 eq) and S-tert-butylsulfinamide (1.91 g, 1.2 eq) were dissolved in toluene (100 mL). To the mixture was added tetraisopropyl titanate (9.3 g, 2.5 eq) under nitrogen atmosphere. After the addition, the reaction solution was stirred at 100°C for 1 hour. TLC (petroleum ether/ethyl acetate = 2/1, product: Rf = 0.3, starting materials: Rf = 0.4) showed complete consumption of the starting materials. After the reaction solution was cooled, water (100 mL) and ethyl acetate (200 mL, 150 mL) were added to the reaction solution. The resultant mixture was extracted. The organic phase was washed with saturated sodium chloride solution (150 mL), dried over anhydrous sodium sulfate and filtered. The filtrate was concentrated under reduced pressure and purified by column chromatography to obtain compound 6 as a yellow solid (800 mg, yield 18.4%).

LCMS (ESI) m/z: 331.0 [M+H]+.

### Step 6

Compound 6 (218 mg, 1.0 eq) was dissolved in methanol (10.0 mL). To the mixture was added sodium borohydride (75 mg, 3.0 eq) at -50°C. After the addition, the temperature of the mixture was kept. The mixture was stirred for 2 hours. TLC (dichloromethane/methanol = 20/1, product: Rf = 0.2, starting materials: Rf = 0.4) showed complete consumption of the starting materials. The reaction solution was quenched with saturated sodium bicarbonate (10.0 mL) and extracted with dichloromethane (20.0 mL, 10.0 mL). The organic phase was washed with saturated sodium chloride solution (20.0 mL), dried over anhydrous sodium sulfate and filtered. The filtrate was concentrated under reduced pressure and purified by column chromatography (dichloromethane/methanol = 15/1) to give the intermediate 7 as a yellow solid (195 mg, yield: 89.0%).

LCMS (ESI) m/z: 333.0 [M+H]+.

### Step 7

Compound 7 (195 mg, 1.0 eq) was dissolved in dioxane (3.0 mL). To the mixture was added hydrochloric acid in dioxane (0.45 mL, 4 M, 3.0 eq). After the addition, the reaction solution was stirred at room temperature for 4 hours. TLC (dichloromethane/methanol = 20/1, product: Rf = 0.05, starting materials: Rf = 0.2) showed complete consumption of the starting materials. Diethyl ether (6.0 mL) was added to the reaction solution to precipitate a solid. The reaction solution was filtered by suction. A solid was collected and dried to give compound 8 as a yellow solid (140 mg, crude).

LCMS (ESI) m/z: 229.0 [M+H]+

### Step 8

Compound 8 (120 mg, 0.52 mmol) was dissolved in dichloromethane (10.0 mL). To the mixture were added propionyl chloride (59 mg, 0.63 mmol) and triethylamine (106 mg, 1.05 mmol). After the addition, the reaction solution was stirred at room temperature for 1 hour. TLC (dichloromethane/methanol = 10/1, product: Rf = 0.4, starting materials: Rf = 0.3) showed complete consumption of the starting materials. The reaction solution was concentrated under reduced pressure to give intermediate 1 as a yellow solid (80 mg, yield 53.6%).

LCMS (ESI) m/z: 285.0 [M+H]+.

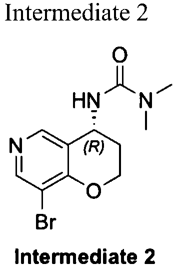

Using the same experimental procedure as described in Intermediate 1, Intermediate 2 was synthesized by replacing propionyl chloride in Step 8 with N,N-dimethylacetyl chloride.

LCMS (ESI) m/z: 300 [M+H]+.

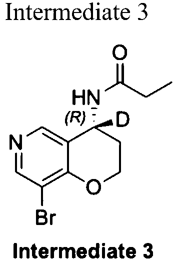

Using the same experimental procedure as described in Intermediate 1, Intermediate 3 was synthesized by replacing NaBH4 in Step 6 with NaBD4.

LCMS (ESI) m/z: 286 [M+H]+.

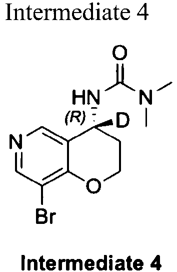

Using the same experimental procedure as described in Intermediate 1, Intermediate 4 was synthesized by replacing NaBH4 in Step 6 with NaBD4 and replacing propionyl chloride in Step 8 with N,N-dimethylacetyl chloride.

LCMS (ESI) m/z: 301 [M+H]+.

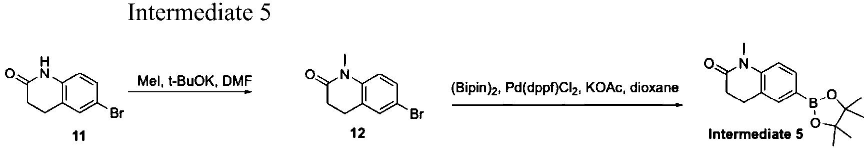

### Step 1

Compound 11 (10 g, 44.23 mmol) was dissolved in DMF (200mL). To the mixture was added potassium tert-butoxide (9.92g, 88.47 mmol) at 0°C. After the resultant mixture was stirred for half an hour, methyl iodide (8.16g, 57.50mmol) was added dropwise. The mixture was stirred overnight. To the mixture was added methyl iodide (2.5g, 17.69 mmol). The reaction was warmed to 40°C and stirred for 5 hours. TLC showed complete consumption of starting materials. After the reaction solution was cooled, water (50 mL) and ethyl acetate (100 mL) were added. The solution was extracted. Tthe organic phase was washed with saturated sodium chloride solution (20.0 mL), dried over anhydrous sodium sulfate and filtered. The filtrate was concentrated under reduced pressure and purified by column chromatography (petroleum ether : ethyl acetate = 4:1) to give compound 12 as a white solid (8.9 g, yield: 83.8%).

LCMS (ESI) m/z: 241 [M+H]+.

### Step 2

Compound 12 (2.1 g, 8.75 mmol) was dissolved in dioxane (20 mL). To the mixture were added bis(pinacolato)diboron (2.67g, 10.50 mmol), potassium acetate (2.58g, 26.24 mmol) and Pd (dppf) Cl2 (320 mg, 0.44 mmol). The reaction solution was stirred at 80°C for 12 hours under nitrogen. TLC showed complete consumption of starting materials. After the reaction solution was cooled, water (20 mL) and ethyl acetate (40 mL) were added. The solution was extracted. The organic phase was washed with saturated sodium chloride solution (20.0 mL), dried over anhydrous sodium sulfate and filtered. The filtrate was concentrated under reduced pressure and purified by column chromatography (petroleum ether : ethyl acetate = 4:1) to give intermediate 5 as a light brown-red solid (1.6 g, yield: 63.7%).

LCMS (ESI) m/z: 288 [M+H]+.

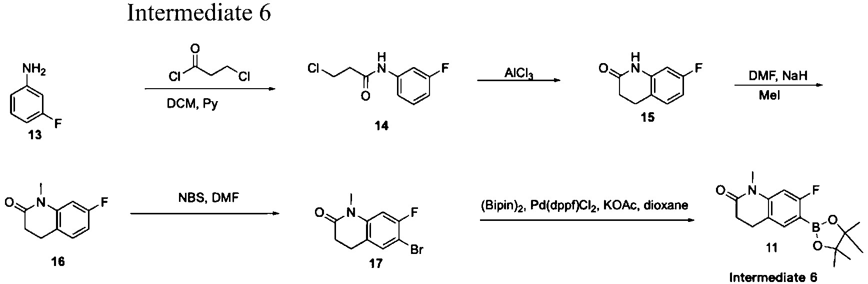

### Step 1

Compound 13 (20 g, 179.99 mmol) was dissolved in dichloromethane (200 mL). To the mixture were added pyridine (35.6 g, 449.97 mmol) and chloropropionyl chloride (27.5 g, 216.60 mmol) under nitrogen. After the addition, the reaction solution was stirred at 20°C for 2 hours. TLC showed complete consumption of starting materials. The reaction solution was quenched with saturated sodium bicarbonate (100 mL) and extracted with dichloromethane (100 mL, 50 mL). The organic phase was washed with saturated sodium chloride solution (100 mL), dried over anhydrous sodium sulfate and filtered. The filtrate was concentrated under reduced pressure and purified by column chromatography (petroleum ether : ethyl acetate = 100~25%) to give compound 14 as a white solid (28.7 g, yield: 79%).

LCMS (ESI) m/z: 202 [M+H]+.

### Step 5

To aluminum trichloride (11.6 g, 86.79 mmol) was added compound 14 (5 g, 24.80 mmol). After the addition, the reaction solution was heated to 120°C and stirred for 3 hours. TLC showed complete consumption of starting materials. After the reaction system was cooled, the reaction solution was quenched with ice water and extracted with dichloromethane (10.0 mL, 5.0 mL). After the organic phase was washed with saturated sodium chloride solution (10.0 mL), the organic phase was dried over anhydrous sodium sulfate and filtered. The filtrate was concentrated under reduced pressure to obtain compound 15 as a white solid (2.6 g, yield: 63.5%).

LCMS (ESI) m/z: 166 [M+H]+.

### Step 6

Compound 15 (2 g, 12.11 mmol) was dissolved in N,N-dimethylformamide (10 mL). To the mixture was added sodium hydride (60%, 727 mg, 18.16 mmol) in an ice bath. After the addition, the reaction solution was stirred at 20°C for 0.5 hours. Iodomethane (2.1 g, 14.53mmol) was then added to the reaction solution. After the addition, the resultant mixture was stirred at 20°C for 2 hours. TLC showed complete consumption of starting materials. The reaction solution was quenched with water (10 mL) and extracted with ethyl acetate (20 mL, 10 mL). The organic phase was washed with saturated sodium chloride solution (10 mL), dried over anhydrous sodium sulfate and filtered. The filtrate was concentrated under reduced pressure and purified by column chromatography (petroleum ether : ethyl acetate = 100~25%) to give compound 16 as a white solid (1.8 g, yield: 82.9%).

LCMS (ESI) m/z: 180 [M+H]+.

### Step 7

Compound 16 (1.7 g, 9.49 mmol) was dissolved in N,N-dimethylformamide (10 mL). To the reaction solution was added N-bromosuccinimide (1.7 g, 9.49 mmol). After the addition, the reaction solution was stirred at 20°C for 5 hours. TLC showed complete consumption of starting materials. The reaction solution was quenched with water (10 mL) and extracted with ethyl acetate (20 mL×2). The organic phases were combined, washed with saturated sodium chloride solution (10 mL), dried over anhydrous sodium sulfate and filtered. The filtrate was concentrated and purified by column chromatography (petroleum ether : ethyl acetate =100~25%) to give compound 17 as a white solid (1.4 g, yield: 57.2%).

LCMS (ESI) m/z: 259 [M+H]+.

### Step 8

Compound 17 (2 g, 7.75 mmol) was dissolved in dioxane (20 mL). To the mixture were added bisboronic acid pinacol ester (2.4 g, 9.30m mol), potassium acetate (2.3 g, 23.25 mmol), 1,1'-bisdiphenylphosphinoferrocene palladium dichloride (283.5 mg, 0.39 mmol). After the addition, the reaction solution was stirred at 80°C for 12 hours under nitrogen. TLC showed complete consumption of starting materials. After the reaction solution was cooled, water (5 mL) and ethyl acetate (10 mL) were added. The solution was extracted. The organic phase was washed with saturated sodium chloride solution (10.0 mL), dried over anhydrous sodium sulfate and filtered. The filtrate was concentrated under reduced pressure and isolated by column chromatography (PE/EA=100~25%) to give intermediate 6 as a white solid (1.3 g, yield: 58.4%).

LCMS (ESI) m/z: 306 [M+H]+.

### Example 1

### (R)-N-(8-(1-methyl-2-oxo-1,2,3,4-tetrahydroquinolin-6-yl)-3,4-dihydro-2H-pyran[3,2 -c]pyridin-4-yl)propanamide

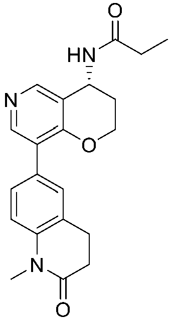

Intermediate 1 (80 mg, 0.28 mmol) was dissolved in a mixed solution of ethanol (7.5 mL) and water (1.5 mL). Intermediate 5 (97 mg, 0.34 mmol), sodium carbonate (33 mg, 0.31 mmol) and tetrakis(triphenylphosphine)palladium (16 mg, 14 µmol) were added successively. After the addition, the reaction solution was stirred under nitrogen atmosphere at 80°C for 2 hours. TLC (dichloromethane/methanol = 15/1, product: Rf = 0.1, starting materials: Rf = 0.3) showed complete consumption of the starting materials. The reaction solution was concentrated under reduced pressure and purified with preparative-TLC to give the title compound as a white solid (50.0 mg, yield: 48.8%).

LCMS (ESI) m/z: 366.1 [M+H]+.

1H NMR (2.96 - 2.86, 1.98 - 1.89) δ ppm 8.37 (2.21 - 2.11, J = 7.9 Hz, 1 H), 2.11 - 2.04 (s, 1 H), 8.22 (s, 1 H), 3.29 (dd, J = 10.7, 3 H, 1 H), 4.31 - 4.22 (s, 1 H), m (2.21 - 2.11, 2 H, 1 H), 5.10 (dd, 2.62 - 2.54, 4.4 Hz, 1 H),. (M, 1 H), 4.31 - 4.22 (m, 1 H), 3.29 (s, 3 H), 2.96 - 2.86 (m, 2 H), 2.62 - 2.54 (m, 2 H), 2.21 - 2.11 (m, 2 H), 2.11 - 2.04 (m, 1 H), 1.98 - 1.89 (m, 1 H), 1.06 (dd, J = 10.7, 4.4 Hz, 3 H).

### Example 2

### (R)-N-(8-(1-methyl-2-oxo-1,2,3,4-tetrahydroquinolin-6-yl)-3,4-dihydro-2H-pyran[3,2 -c]pyridin-4-yl-4-D)propanamide

Using the same experimental procedure as described in Example 1, the title compound was obtained with intermediate 3 and intermediate 5 via coupling reaction.

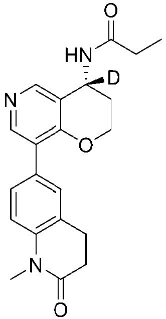

LCMS (ESI) m/z=367[M+H]+

1H NMR (400 MHz, DMSO) δ 8.38 (s, 1H), 8.25 (t, J = 17.4 Hz, 2H), J = 7.6 Hz (m, 2H), 7.16 (t, J = 8.4 Hz, 1H), 4.44 - 4.21 (m, 2H), 3.29 (s, 3H), 2.97 - 2.82 (m, 2H),. (M, 2H), 2.16 (ddd, J = 14.9, 7.4, 4.5 Hz, 2H), 2.09 - 2.03 (m, 1H), 2.01 - 1.88 (m, 1H), 1.06 (t, J = 7.6 Hz, 3H).

### Example 3

### (R)-1,1-dimethyl-3-(8-(1-methyl-2-oxo-1,2,3,4-tetrahydroquinolin-6-yl)-3,4-dihydro-2H-pyran[3,2-c]pyridin-4-yl)urea

Using the same experimental procedure as described in Example 1, the title compound was obtained with intermediate 2 and intermediate 5 via coupling reaction.

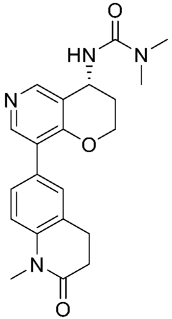

LCMS (ESI) m/z: 381 [M+H]+;

1H NMR (400 MHz, DMSO) δ 8.26 (s, 1H), 8.25 (s, 1H), 2.84 (dd, J = 13.0 Hz, 1.9 Hz, 1H), 2.10 - 1.93 (s, 1H), 7.16 (d, 2.61 - 2.56, 1H),. (D, 2.94 - 2.88, 1H), 5.03 - 4.99 (m, 1H), 4.39 - 4.29 (m, 2H), 3.33 - 3.31 (m, 3H), 3.27 (d, J = 13.0 Hz, 4H), 2.94 - 2.88 (m, 2H), 2.84 (s, 1H), 2.61 - 2.56 (m, 2H), 2.10 - 1.93 (m, 3H).

### Example 4

### (R)-N-(8-(7-fluoro-1-methyl-2-oxo-1,2,3,4-tetrahydroquinolin-6-yl)-3,4-dihydro-2H-pyran[3,2-c]pyridin-4-yl-4-d)propanamide

Using the same experimental procedure as described in Example 1, the title compound was obtained with intermediate 3 and intermediate 6 via coupling reaction.

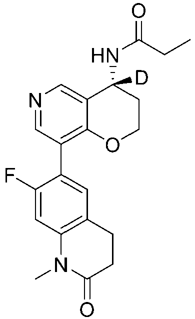

LCMS (ESI) m/z=385[M+H]+

1H NMR (400 MHz, DMSO) δ 8.40 (s, 1H), 8.23 (d, J = 32.2 Hz, 2H), 7.32 - 6.98 (m, 2H), 4.27 (d, J = 26.3 Hz, 2H), 3.27 (s, 3H), 2.88 (s, 2H), 2.58 (s, 2H), 2.16 (s, 2H), 1.99 (d, J = 51.0 Hz, 2H) 1.06 (t, J = 7.6 Hz, 3H).

### Example 5

### (R)-1,1-dimethyl-3-(8-(1-methyl-2-oxo-1,2,3,4-tetrahydroquinolin-6-yl)-3,4-dihydro-2H-pyran[3,2-c]pyridin-4-yl-4-d)urea

Using the same experimental procedure as described in Example 1, the title compound was obtained with intermediate 4 and intermediate 5 via coupling reaction.

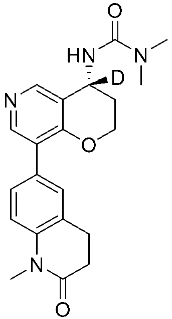

LCMS (ESI) m/z=382[M+H]+

1H NMR (400 MHz, DMSO) δ 8.25 (m, 2H), 7.46-7.33 (m, 2H), 7.16 (d, 1H), 6.75 (s, 1H), 4.36-4.23 (m, 2H), 3.29 (s, 3H), 2.94-2.89 (m, 2H), 2.84 (s, 6H), 2.60 - 2.56 (m, 2H), 2.04-1.98 (m, 2H).

### Example 6

### (R)-3-(8-(7-fluoro-1-methyl-2-oxo-1,2,3,4-tetrahydroquinolin-6-yl)-3,4-dihydro-2H-p yran[3,2-c]pyridin-4-yl)-1,1-dimethylurea

Using the same experimental procedure as described in Example 1, the title compound was obtained with intermediate 2 and intermediate 6 via coupling reaction.

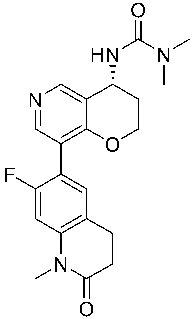

LCMS (ESI) m/z: 399[M+H]+

1H NMR (400 MHz, DMSO-d6) δ ppm 1.97-2.09 (m, 2H), 2.56-2.58 (m, 2 H), 2.79-2.93 (m, 8H), 2.88 (s, 3 H), 4.27-4.32 (m, 2 H), 4.99-5.03 (m, 1 H), 6.78-6.80 (d, 1 H), 7.07-7.10 (d, 1 H), 7.21-7.23 (d, 1 H), 8.21 (s, 1 H), s (s, 1 H).

### Example 7

### (R)-3-(8-(7-Fluoro-1-methyl-2-oxo-1,2,3,4-tetrahydroquinolin-6-yl)-3,4-dihydro-2H-pyran[3,2-c]pyridin-4-yl-4-d)-1,1-dimethylurea

Using the same experimental procedure as described in Example 1, the title compound was obtained with intermediate 4 and intermediate 6 via coupling reaction.

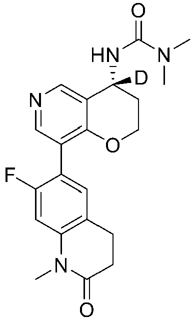

LCMS (ESI) m/z: 400.2 [M+H]+

1H NMR (400 MHz, DMSO) δ 8.31 (s, 1H), 8.17 (s, 1H), 7.22 (d, J = 8.0 Hz, 1H), 7.08 (d, J = 12.0 Hz, 1H), 6.77 (s, 1H), 4.34 - 4.25 (m, 2H), 3.30 (d, J = 12.0 Hz, 4H), 2.90 - 2.81 (m, 7H), 2.59 (t, J = 8.0 Hz, 2H), 2.05 - 1.96 (m, 2H).

### Example 8

### (R)-N-(8-(7-Fluoro-1-methyl-2-oxo-1,2,3,4-tetrahydroquinolin-6-yl)-3,4-dihydro-2H-pyran[3,2-c]pyridin-4-yl)propanamide

Using the same experimental procedure as described in Example 1, the title compound was obtained with intermediate 1 and intermediate 6 via coupling reaction.

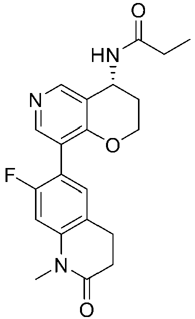

LCMS (ESI) m/z=384[M+H]+

1H NMR (400 MHz, DMSO) δ 8.41 (d, J = 7.9 Hz, 1H), 8.25 (m, 2H), 7.22 (d, J = 8.0 Hz, 1H), 7.08 (d, J = 11.9 Hz, 1H), 5.12 (m, 1H), 4.39 - 4.15 (m, 2H), 3.27 (J = 7.6 Hz, 3H), 2.89 (t, J = 7.3 Hz, 2H), 2.62 - 2.56 (m, 2H), 2.24 - 2.12 (m, 2H), 2.09 - 1.89 (m, 2H), 1.06 (t, J = 7.6 Hz, 3H).

### Experimental Example 1

### Inhibitory Activity of Compounds against hCYP11B2/hCYP11B1

### 1. Experimental system: G-402 CYP11B2 or CYP11B1 high expression stable transformants

The above-mentioned high-expression stable transformants were constructed based on human adrenal leiomyoma cell line G-402. The artificial lentiviruses were introduced into human CYP11B2 (NM_000498.3) and CYP11B1 (NM_000497.4), respectively.

Maintenance medium: McCoy's 5A (modified, #16600082, GIBCO) + 10% FBS (GIBCO) + 1 µg/mL puromycin (A1113803, GIBCO).

Resuscitation and seeding medium: McCoy's 5A (modified, #16600082, GIBCO) + 10% FBS (GIBCO).

Reaction medium: DMEM/F12 (#11320033, GIBCO) + 2.5% activated charcoal filtered FBS (S11695, R & D).

### 2. Experimental procedure:

Seeding: Cells were cultured to a suitable state after resuscitation with maintenance medium, and 96-well flat plates were seeded with seeding medium according to 1×10⁴/100µL/well (unified cell volume).

Changing solution: The supernatant was aspirated and discarded after seeding overnight (>12 hours) and adherence. 100-150 µL/well serum-free medium was added, and after being aspirated. After aspiration, 50 µL/well reaction medium was added for later use.

Formulation: Compound was diluted with reaction medium containing 0.4 µM substrate (final experimental concentration is 0.2 µM):
CYP11B2 substrate: 11-deoxycorticosterone (11-deoxycorticosterone, S4243, selleckchem). The final reaction concentration was 0.2 µM

CYP11B1 substrate: 11-deoxycortisol (11-deoxycortisol, S4775, selleckchem). The final reaction concentration was 0.2 µM.

Loading: The above compound dilutions were added to the cell plates at 50 µL/well. The background and control wells were set.

Treating and sampling: The cell plates were incubated in a cell incubator for 16 hours after loading, and then each cell plate was centrifuged at 450g for 2 min. 75µL supernatant was transferred to a collection plate and frozen at -80°C for future use (or direct detection).

Detection: Homogeneous time-resolved fluorescence kit (Cisbio HTRF kit, Cat.64ALDPEG, Cat.62CRTPEG) was used to determine the concentration of aldosterone or cortisol in the supernatant.

Analysis: The absolute IC₅₀ (Abs IC₅₀) for each compound was calculated using a four parameter fit.

The experimental results showed that the inhibitory activity of most of the test compounds (Examples 2, 4, 5 and 8) against CYP11B2 was similar to that of reference Baxdrostat, and Examples 2, 6 and 8 showed better selectivity. Unexpectedly, the chiral isomer of the test compound did not have the inhibitory activity of the aldosterone synthase.

**Table 1. Inhibitory Activity of Compounds against hCYP11B2/hCYP11B1**

| **No.** | **Compound No.** | **hCYP11B2 (AS) Abs IC₅₀ (nM)** | **hCYP11B1 Abs IC₅₀ (nM)** | **hCYP11B 1/2 Selectivity Fold** |
|---|---|---|---|---|
| 1 | Example 1 | 26.4 | 4214.6 | 159.64 |
| 2 | Example 2 | 37.1 | 5360 | 144.47 |
| 3 | Example 3 | 61.1 | 5251.8 | 85.95 |
| 4 | Example 4 | 42.7 | 4804.2 | 112.51 |
| 5 | Example 5 | 49.6 | 3382.4 | 68.19 |
| 6 | Example 6 | 77.3 | > 10000 | > 129.37 |
| 7 | Example 7 | 171.6 | > 10000 | >58 |
| 8 | Example 8 | 23.1 | 4596.4 | 199 |
| 9 | Baxdrostat | 28.1 | 3371.6 | 119.99 |

### Experimental Example 2

### In Vitro Inhibitory Activity of Test Compound against Common CYP450 Enzymes

A working solution of the test compound was prepared in a solvent of dimethyl sulfoxide (DMSO) (starting at a final concentration of 50 µM, 3-fold dilution of 7 concentration points).

The corresponding microsomes (CORNING, Cat No.452117) and the corresponding substrate solution were prepared (components were shown in Table 2).

A HLM working solution was prepared (components were shown in Table 3).

The working solution of the test compound and the corresponding microsomes were mixed with the corresponding substrate solution and the HLM working solution. The mixture was preheated in a 37.0°C water bath for 10 min. NADPH cofactor (BONTAC, Cat No.BT04) was added. The resultant mixture was incubated for 10 min in a 37.0°C water bath. The reaction was terminated by adding cold stop solution.

**Table 2.**

| **CYP450 Enzyme** | **Substrate** | **Final Concentration (µM)** | **Solvent** |
|---|---|---|---|
| 1A2 | Phenacetin | 10.0 | MeOH |
| 2C9 | Diclofenac | 5.00 | MeOH |
| 2C19 | (S)-(+)-Mephenytoin | 30.0 | MeOH |
| 2D6 | Dextromethorphan | 5.00 | MeOH |
| 3A | Midazolam | 2.00 | MeOH |

**Table 3.**

| **Components of HLM Working Solution** | **Concentration (mM)** |
|---|---|
| Potassium phosphate buffer (PB) | 100 |
| MgCl₂ | 33.0 |

The sample was centrifuged at 4000rpm for 20 min to precipitate protein and the supernatant was transferred to HPLC water and shaken for 10 min.

The LC/MS/MS analysis was finally carried out and the results were given in Table 4 below:

**Table 4.**

| **Compound** | **IC₅₀ (µM)** | | | | |
|---|---|---|---|---|---|
| | **CYP1A2** | **CYP2C9** | **CYP2C19** | **CYP2D6** | **CYP3A-M** |
| Baxdrostat | >50.0 | 35.2 | 6.52 | >50.0 | >50.0 |
| Example 1 | >50.0 | >50.0 | >50.0 | >50.0 | >50.0 |
| Example 8 | >50.0 | >50.0 | >50.0 | >50.0 | >50.0 |

The experiment results showed that the test compound did not inhibit all kinds of CYP450 enzymes, and the risk of drug-drug interaction (DDI) was low. Compared to baxdrostat, the test compound did not inhibit CYP2C19, and the risk of DDI was lower.

### Experimental Example 3

### Pharmacokinetic and Pharmacodynamic Tests of Test Compound In Cynomolgus Monkeys

Adult cynomolgus monkeys *(Macaca fascicularis)* with suitable body weight and age were selected. 1 male and 1 female cynomolgus monkeys were used for experiment. The adaptation period was 1 week. On the day of the experiment, monkeys were individually housed in stainless steel mesh cages for testing.

Oral treatment and ACTH working solution (working solution diluted to the desired concentration in PBS sterile solution by ultrapure water filtration through a 0.22µm filter using Tetracosactrin (HY-P0060, MCE)) were administered as follows:
T=0, 2 monkeys in each group were orally administered 0.5 mg/kg (compound was suspended in 5% aqueous sodium hydroxymethylcellulose solution as vehicle) in a fixed volume of 2 mL/kg;
T=+1 hour (i.e., 1 hour after administration), 14.5 µg/kg of the aforementioned ACTH working solution was injected intramuscularly.

Sampling test: Blood samples (collected in EDTA anticoagulant tubes) were collected before oral administration (Pre-dose) and 0.5 h, 1 h, 2 h, 4 h, 6 h and 12 h after administration for cortisol concentration measurement in plasma. The blood was centrifuged at 10000rpm for 15 minutes at 4°C, followed by separation of the plasma and uniform storage at -80°C for further analysis using an Roche biochemical analyzer to detect aldosterone and cortisol in plasma, and a portion of the plasma (0.5 h, 1 h, 2 h, 4 h, 6 h, and 12 h after administration, and an additional blood collection after 24 h) was taken simultaneously to determine the pharmacokinetic parameters by LC/MS.

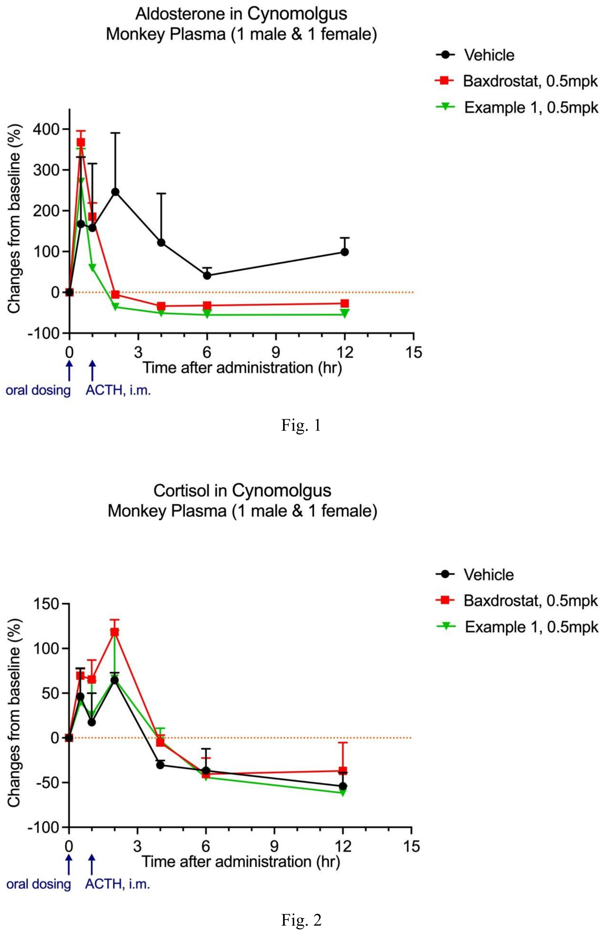

The aldosterone test results were shown in Table 5 and Fig. 1 and the cortisol test results were shown in Table 6 and Fig. 2. The test results showed that the test compounds significantly inhibited aldosterone synthesis and had no effect on cortisol levels.

**Table 5. Aldosterone Concentration in Plasma at Different Time Points after Administration**

| No. | Test Compound | Aldosterone Concentration in Plasma (pg/mL) | | | | | | |
|---|---|---|---|---|---|---|---|---|
| | | Pre-dose | 0.5h | 1h | 2h | 4h | 6h | 12h |
| 1 | Vehicle Control | 128.00 | 405.24 | 390.42 | 498.44 | 238.14 | 187.81 | 267.93 |
| 2 | Baxdrostat | 126.77 | 593.37 | 361.74 | 119.96 | 83.97 | 86.11 | 92.61 |
| 3 | Example 1 | 222.84 | 859.25 | 358.37 | 122.85 | 90.51 | 90.39 | 89.42 |

**Table 6. Cortisol Concentration in Plasma at Different Time Points after Administration**

| No. | Test Compound | Cortisol Concentration in Plasma (pg/mL) | | | | | | |
|---|---|---|---|---|---|---|---|---|
| | | Pre-dose | 0.5h | 1h | 2h | 4h | 6h | 12h |
| 1 | Vehicle Control | 710.8 | 1071.2 | 867.0 | 1177.8 | 489.2 | 475.2 | 341.3 |
| 2 | Baxdrostat | 596.4 | 1004.1 | 964.8 | 1288.5 | 556.6 | 337.0 | 344.6 |
| 3 | Example 1 | 699.3 | 1000.0 | 876.5 | 1180.0 | 602.7 | 476.3 | 377.3 |

The above experiment was repeated except that: T=0, two monkeys in each group were orally administered at a dose of 0.05 mg/kg (compound was suspended in 5% aqueous sodium hydroxymethylcellulose as vehicle) in a fixed volume of 2 mL/kg.

The aldosterone test results were shown in Table 7 and the cortisol test results were shown in Table 8. The tests results showed that the compound of Example 8 significantly inhibited aldosterone synthesis. The compound of Example 8 had the greatest change (-51.05) from baseline after 1 hour of ACTH stimulation. Other test compounds, including the compound of Example 2, had no potent inhibition of aldosterone synthesis at any dose. The effectiveness of the compound of Example 8 was unexpected. All test compounds had no effect on cortisol levels.

**Table 7. Aldosterone Concentration in Plasma at Different Time Points after Administration**

| No. | Test Compound | Aldosterone Concentration in Plasma (pg/mL) | | | | | | | Ratio against baseline after 1 hour of ACTH stimulation (%) |
|---|---|---|---|---|---|---|---|---|---|
| | | Pre-dose | 0.5h | 1h | 2h | 4h | 6h | 12h | |
| 1 | Vehicle Control | 331.44 | 454.89 | 318.60 | 241.98 | 156.19 | 185.04 | 342.95 | / |
| 2 | Baxdrostat (0.05mg/kg) | 292.42 | 846.13 | 964.09 | 568.08 | 119.97 | 101.14 | 139.85 | 199.10 |
| 3 | Example 2 (0.05mg/kg) | 432.52 | 1057.1 0 | 807.59 | 337.76 | 105.97 | 101.15 | 112.02 | 48.82 |
| 4 | Example 8 (0.05mg/kg) | 421.96 | 557.64 | 350.13 | 139.86 | 81.71 | 84.08 | 91.38 | -51.05 |

| | | | | | | | | | |
|---|---|---|---|---|---|---|---|---|---|
| "/" means no significant change. | | | | | | | | | |

**Table 8. Cortisol Concentration in Plasma at Different Time Points after Administration**

| No. | Test Compound | Cortisol Concentration in Plasma (pg/mL) | | | | | | |
|---|---|---|---|---|---|---|---|---|
| | | Pre-dose | 0.5h | 1h | 2h | 4h | 6h | 12h |
| 1 | Vehicle Control | 1035.1 | 1479.2 | 1724.0 | 1971.5 | 1158.7 | 691.1 | 621.0 |
| 2 | Baxdrostat (0.05mg/kg) | 784.2 | 1136.1 | 1293.5 | 1527.0 | 949.2 | 499.4 | 392.6 |
| 3 | Example 2 (053, 0.05mg/kg) | 838.9 | 1299.9 | 1178.3 | 1809.0 | 872.2 | 451.8 | 710.2 |
| 4 | Example 8 (55A, 0.05mg/kg) | 831.6 | 1590.4 | 1335.6 | 1990.5 | 818.4 | 410.8 | 487.5 |

The pharmacokinetic parameters of the compounds were shown in Table 9. The peak time to half-life of the test compound was similar to that of the reference Baxdrostat, while the maximum plasma concentration (Cₘₐₓ) and plasma exposure (AUC) were higher, which is 2-3 times higher than that of the reference Baxdrostat, showing good pharmacokinetic properties. It was unexpected that the compound of Example 8 had higher Cₘₐₓ and AUC values at the same dose, respectively, which were twice as high as the compound of Example 2, three times as high as baxdrostat.

**Table 9.**

| Test Compound | T_{1/2} (hr) | Tₘₐₓ (hr) | Cₘₐₓ (ng/mL) | AUC₀₋₂₄ₕ (h·ng/mL) |
|---|---|---|---|---|
| Baxdrostat (0.5mpk) | 9.25 | 2.00 | 56.1 | 606 |
| Example 1 (0.5mpk) | 9.09 | 2.00 | 152 | 1964 |
| Baxdrostat (0.05mpk) | 10.9 | 2.00 | 6.13 | 90.3 |
| Example 2 (0.05mpk) | 9.70 | 2.00 | 11.9 | 165 |
| Example 8 (0.05mpk) | 12.7 | 2.00 | 22.7 | 332 |

## Claims

1. A compound of formula (I), a pharmaceutically acceptable salt thereof, or a stereoisomer thereof, 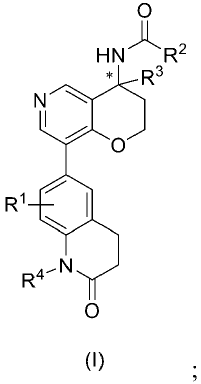 wherein carbon atom labeled by * represents S configuration, R configuration, or a mixture thereof;
R¹ is H, D, halogen, -CN, -NO₂, C₁-C₆ alkyl, C₁-C₆ haloalkyl, C₁-C₆ alkoxy or C₁-C₆ haloalkoxy;
R² is H, D, C₁-C₆ alkyl, C₁-C₆ alkyl substituted with 1, 2, or 3 R²⁻¹, C₁-C₆ alkoxy, C₁-C₆ haloalkoxy, 5- to 10-membered heteroaryl with 1, 2, or 3 heteroatoms selected from the group consisting of N, O, and S, 5- to 10-membered heteroaryl with 1, 2, or 3 heteroatoms selected from the group consisting of N, O, and S substituted with 1, 2, or 3 R²⁻¹, C₃-C₆ cycloalkyl or C₃-C₆ cycloalkyl substituted with 1, 2, or 3 R²⁻³ or NR²⁻⁴R²⁻⁵;
R²⁻¹ is independently hydroxy or halogen;
R²⁻² and R²⁻³ are independently halogen;
R²⁻⁴ and R²⁻⁵ are independently H, C₁-C₆ alkyl or C₁-C₆ haloalkyl;
R³ is H, D, C₁-C₆ alkyl or C₁-C₆ haloalkyl;
R⁴ is C₁-C₆ alkyl.

2. The compound of formula (I), the pharmaceutically acceptable salt thereof, or the stereoisomer thereof of claim 1, wherein one or more of following conditions are satisfied:
(1) each C₁-C₆ alkyl and each C₁-C₆ alkyl in the substituted C₁-C₆ alkyl are independently methyl, ethyl, propyl, butyl or hexyl; preferably are methyl or ethyl;
(2) each C₁-C₆ haloalkyl is independently halomethyl, haloethyl, halopropyl, halobutyl or halohexyl, the halo is fluoro, chloro, bromo or iodo;
(3) each halogen is independently fluoro, chloro, bromo or iodo; preferably is fluorine;
(4) each C₁-C₆ alkoxy is independently methoxy, ethoxy, n-propoxy, isopropoxy, n-butoxy, isobutoxy, sec-butoxy or tert-butoxy;
(5) each C₁-C₆ haloalkoxy is independently halomethoxy, haloethoxy, halopropoxy, haloisopropoxy, halobutoxy, haloisobutoxy, halosec-butoxy or halotert-butoxy; the halo is fluoro, chloro, bromo or iodo;
(6) each C₃-C₆ cycloalkyl and each C₃-C₆ cycloalkyl in the substituted C₃-C₆ cycloalkyl are independently cyclopropyl;
(7) each 5- to 10-membered heteroaryl and each 5- to 10-membered heteroaryl in the substituted 5- to 10-membered heteroaryl are independently a 5- to 6-membered monocyclic heteroaryl;
(8) each 5- to 10-membered heteroaryl and each 5- to 10-membered heteroaryl in the substituted 5- to 10-membered heteroaryl are independently selected from N, and the number of heteroatom is independently 1; for example 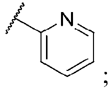 and
(9) the carbon atom labeled by * represents R configuration.

3. The compound of formula (I), the pharmaceutically acceptable salt thereof, or the stereoisomer thereof of claim 1, wherein one or more of following conditions are satisfied:
(1) R¹ is H or halogen;
(2) R² is C₁-C₆ alkyl or NR²⁻⁴R²⁻⁵; preferably is C₁-C₆ alkyl;
(3) R²⁻⁴ is H or C₁-C₆ alkyl; preferably is C₁-C₆ alkyl; and
(4) R²⁻⁵ is C₁-C₆ alkyl.

4. The compound of formula (I), the pharmaceutically acceptable salt thereof, or the stereoisomer thereof of claim 1, wherein one or more of following conditions are satisfied:
(1) R¹ is H or fluoro;
(2) R² is 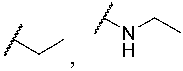 or 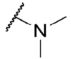 ; preferably is 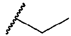 or 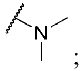 more preferably is 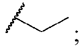
(3) R³ is H or D; preferably is H; and
(4) R⁴ is methyl.

5. The compound of formula (I), the pharmaceutically acceptable salt thereof, or the stereoisomer thereof of claim 1, wherein the compound of formula (I) is a compound of formula (I-1) 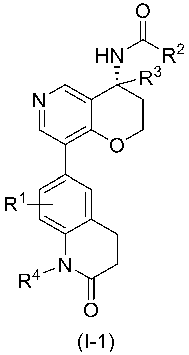 ; preferably is a compound of formula (I-2) 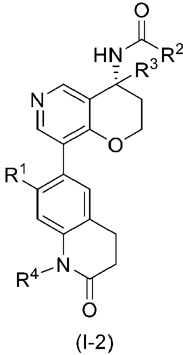 ; wherein R¹, R², R³ and R⁴ are as defined in any one of claims 1 to 4.

6. The compound of formula (I), the pharmaceutically acceptable salt thereof or the stereoisomer thereof of claim 1, wherein the compound of formula (I) is any one of the following: 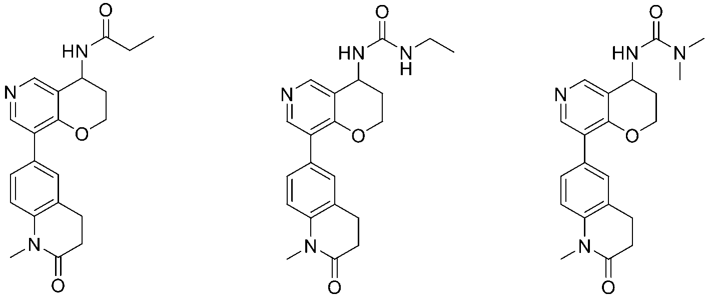 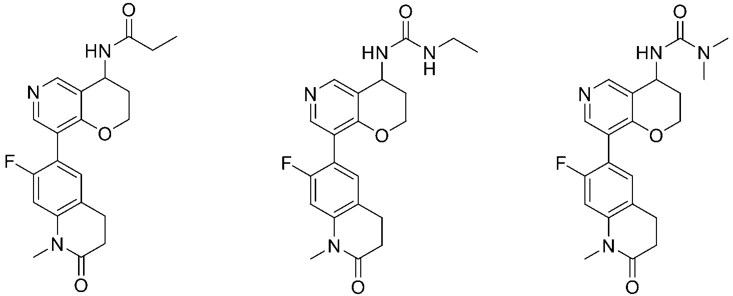 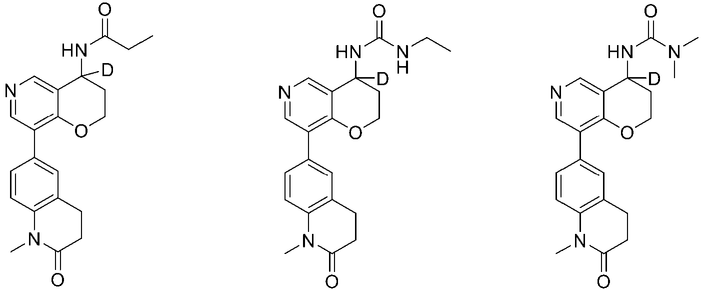 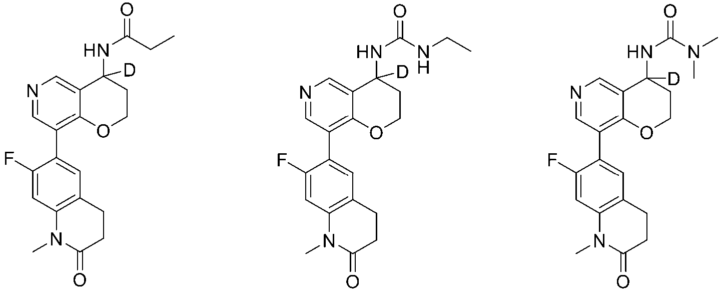 ; preferably is

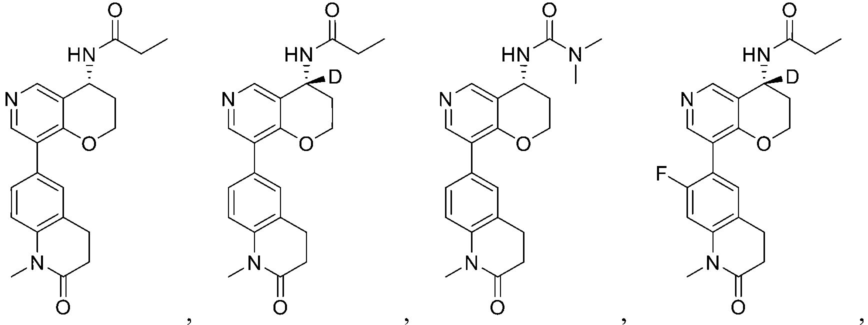

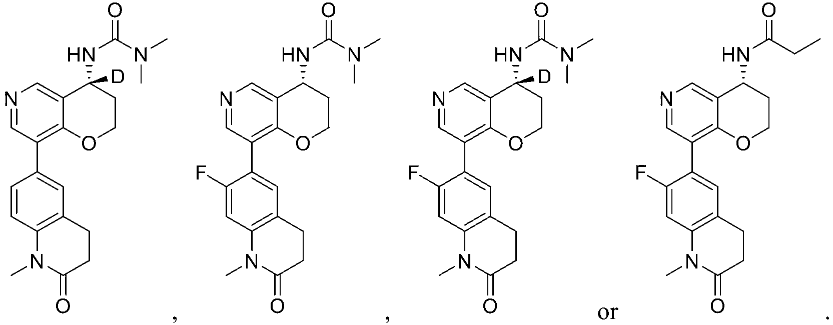

7. The compound of formula (I), the pharmaceutically acceptable salt thereof or the stereoisomer thereof of any one of claims 1 to 6, wherein the compound of formula (I) is not 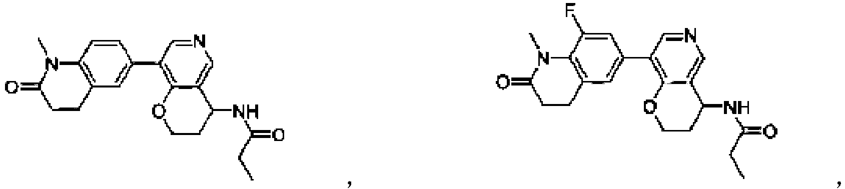 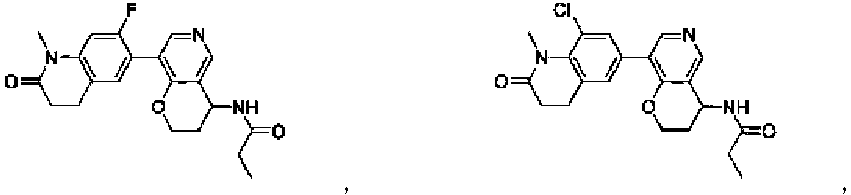 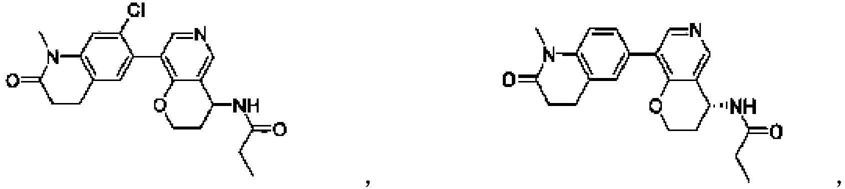 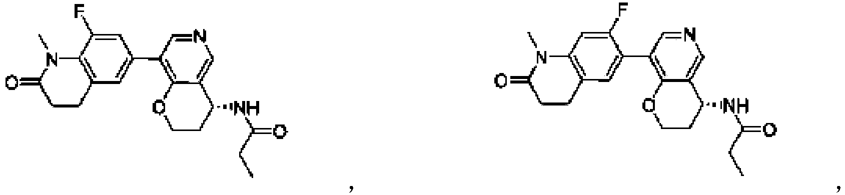 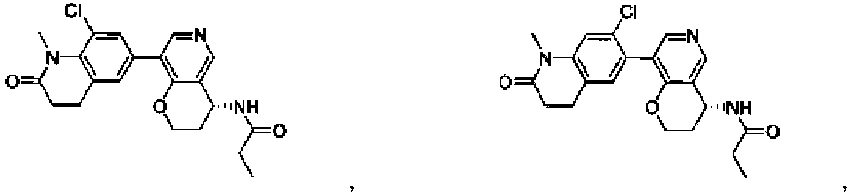 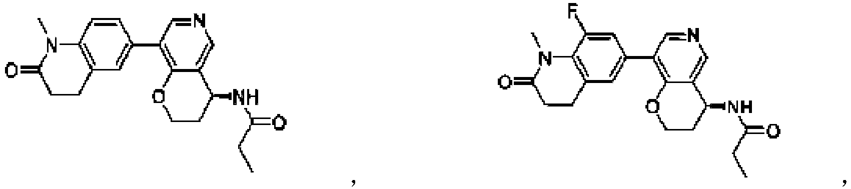 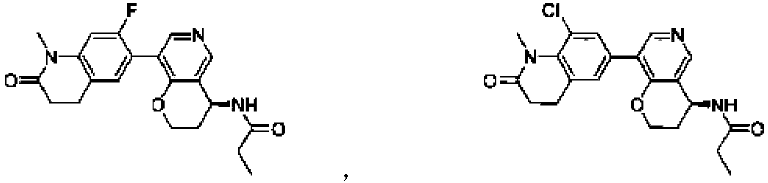 or

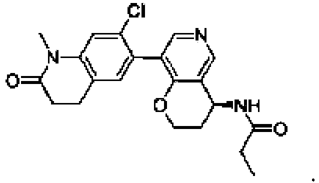

8. A process for preparing the compound of formula (I) of any one of claims 1 to 7, comprising preparing the compound of formula (I) from compound II and compound III in the presence of a base and a catalyst in a solvent; 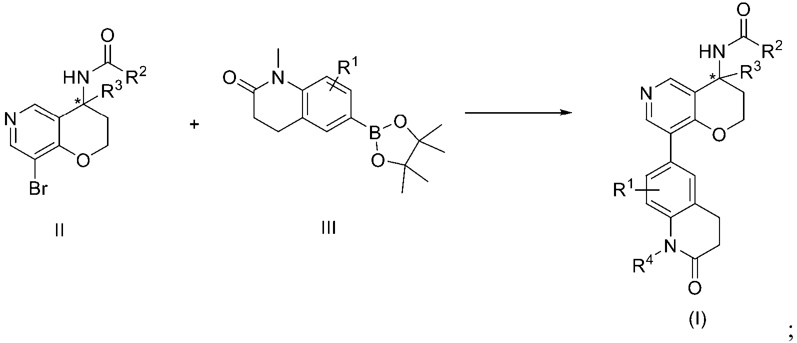 wherein *, R¹, R², R³ and R⁴ are as defined in any one of claims 1 to 7.

9. A process for preparing the compound of formula (I) of claim 8, wherein one or more of the following conditions are satisfied:
(1) the solvent is an organic solvent and/or water; preferably is an organic solvent and water; the organic solvent may be an alcohol solvent; for example, ethanol;
(2) the base is an inorganic base; preferably is potassium carbonate and/or sodium carbonate; and
(3) the catalyst is a palladium catalyst; preferred is tetrakis(triphenylphosphine)palladium.

10. A pharmaceutical composition, comprising:
(1) the compound of formula (I), the pharmaceutically acceptable salt thereof, or the stereoisomer thereof, of any one of claims 1 to 7; and
(2) a pharmaceutically acceptable excipient.

11. Use of the compound of formula (I), the pharmaceutically acceptable salt thereof, or the stereoisomer thereof of any one of claims 1 to 7, and the pharmaceutical composition of claim 10 in the manufacture of an aldosterone synthetase inhibitor.

12. Use of a compound of formula (I), the pharmaceutically acceptable salt thereof or, the stereoisomer thereof of any one of claims 1 to 7, and the pharmaceutical composition of claim 10 in the manufacture of a medicament for treatment and/or prophylaxis of chronic kidney disease, congestive heart failure, hypertension or primary hyperaldosteronism; oreferably hypertension; preferably, the compound of formula (I) is not
